# EUROPÄISCHE PATENTSCHRIFT

(11) **EP 2 085 113 B1**
(45) Veröffentlichungstag und Bekanntmachung des Hinweises auf die Patenterteilung: **25.04.2012**
(21) Anmeldenummer: 09000422.7
(22) Anmeldetag: 14.01.2009
(51) Int. Cl.: A61N 1/36, A61N 1/05

(54) **Implantierbare bipolare Elektrode**
Implantable bipolar electrode
Electrode bipolaire implantable

(30) Priorität: 22.01.2008 DE 102008005378
(43) Veröffentlichungstag der Anmeldung: 05.08.2009
(73) Patentinhaber: Osypka, Peter, 79618 Rheinfelden-Herten (DE)
(72) Erfinder: Osypka, Peter, 79618 Rheinfelden-Herten (DE)
(74) Vertreter: Maucher, Wolfgang

(56) Entgegenhaltungen:
- WO-A-2007/073435
- WO-A-2008/066423
- US-A- 5 324 325
- US-A- 5 716 390
- US-A1- 2002 188 338
- US-A1- 2006 036 306
- US-B1- 7 383 091

## Beschreibung

Die Erfindung betrifft eine implantierbare bipolare Elektrode mit einer an ihrem distalen Ende befindlichen, von einem Schaft oder Schlauch getragenen Kathode und mit einer dazu beabstandeten Anode, wobei die Elektrode am distalen Ende einen Anker zum Befestigen im Gewebe eines Herzens aufweist, wobei die bipolare Elektrode aus zwei unipolaren Elektroden gebildet ist, deren eine an ihrem distalen Ende die Kathode und den oder die Anker aufweist und deren andere an einem zweiten Schaft oder Schlauch die Anode aufweist, wobei der zweite Schaft den die Kathode aufweisenden ersten Schaft in sich aufnimmt und relativ zu diesem in axialer Richtung verstellbar oder verschiebbar ist und zumindest in Gebrauchsstellung Kathode und Anode jeweils elektrisch mit einem bipolaren Stecker verbunden sind.

Eine derartige bipolare Elektrode ist aus der US 2006/0064150 A1 und aus der US 2006/ 0036306 A1 bekannt. Als Anker sind dabei an der mit dem zweiten, außenliegenden Schaft verbundenen Anode Zinken oder biegsame Stifte als Anker vorgesehen, die aber nur dann wirksam werden können, wenn der Kopf dieses Schaftes in ein Gewebe genügend tief eingedrückt werden kann- Solche widerhakenartige Stifte können Kollisionen oder Verhakungen vor allem beim Durchgang durch die Nitralkappe verursachen. Wäre der Anker am distalen Ende der Kathode als Schraubelektrode oder Schraubwendel ausgebildet, könnte die Wandung des oder der Gefäße verletzt werden, durch welche die Elektroden zum Herzen hin verschoben werden muss.

Zwar sind Herzschrittmacherelektroden mit zur Verankerung dienenden Schraubwendeln beispielsweise aus der US 5 716 390 bekannt, die während der Implantation in den Elektrodenschlauch zurückgezogen sind und im Herzen mit Hilfe eines speziellen Mechanismus für die durchzuführende Verankerung axial verschoben werden können, was jedoch einen erheblichen Aufwand bedeutet. Denkbare Verhakungen mit stiftartigen oder widerhakenartigen Ankern werden bisher in Kauf genommen.

Es besteht deshalb die Aufgabe, eine Elektrode der eingangs genannten Art zu schaffen, mit welcher eine problemlose Implantation im Herzen möglich ist, ohne dass es zu Verletzungen durch die Schraubelektrode oder zu Verhakungen mittels stiftartigen Ankern kommen kann.

Zur Lösung dieser Aufgabe ist vorgesehen, dass der zweite Schaft den als Schraubwendel ausgebildeten Anker während der Implantation in sich aufnimmt und umschließt und dass der die Anode aufweisende zweite Schaft zumindest um das Maß axial relativ zu dem ersten Schaft verstellbar ist, welches die Schraubwendel gegenüber dem distalen Ende des ersten Schafts hat.

In Gebrauchsstellung sind also beide Pole, also Kathode und Anode, die ihrerseits jeweils auch mehrfach vorhanden sein könnten, mit einem bipolaren Stecker zum Verbinden mit einem Herzschrittmacher verbunden, so dass die gesamte Anordnung eine bipolare Elektrode ist. Während der Implantation ist es dabei möglich, den die Anode tragenden Schaft oder Schlauch so weit verschoben vorzusehen, dass die an dem die Kathode aufweisenden Schaft oder Schlauch befindliche Verankerung davon umschlossen ist, so dass es während der Implantation weder zu Verletzungen durch eine schraubenförmige Verankerung noch zu einer Verhakung von stiftartigen Verankerungen kommen kann. Hat die Elektrode mit ihrem distalen Ende ihr Ziel erreicht, kann der die Anode tragende Schaft in seine übliche Gebrauchsstellung zurückgezogen und die Verankerung im Inneren des Herzens durchgeführt werden, wobei aufwendige Mechanismen zum axialen Verstellen einer Schraubverankerung nicht erforderlich sind, sondern vermieden werden.

Weil der die Anode aufweisende zweite Schaft um das Maß axial relativ zu dem ersten Schaft verstellbar oder zurückziehbar ist um welches der als Schraubwendel ausgebildete Anker gegenüber dem distalen Ende des ersten Schafts vorsteht, kann die Länge dieser als Verankerung dienenden Schraubwendel bei der Verankerung voll ausgenutzt werden. Die Schraubwendel kann aber während der Implantation vollständig von dem zweiten Schlauch oder Schaft umschlossen werden, sodass Verletzungen der Gefäße vermieden werden.

Eine zweckmäßige Ausgestaltung der Erfindung kann vorsehen, dass der die Anode tragende zweite Schaft den die Kathode aufweisenden ersten Schaft konzentrisch umschließt. Die Schäfte können also einen etwa kreisrunden Querschnitt haben, was erforderlichenfalls auch noch eine Verdrehung der gesamten Elektrode oder auch eine Relativverdrehung der beiden Schäfte zueinander ermöglicht oder erleichtert.

Eine weitere vorteilhafte Ausgestaltung der Erfindung kann darin bestehen, dass der die Anode tragende zweite Schaft an seinem distalen Ende benachbart zu der Anode ein hülsenartiges Isolierstück, vorzugsweise eine Verlängerung des schlauchartigen Schafts relativ zu der Anode aufweist, welches gegenüber der Anode vorsteht. Dadurch ist es möglich, in Gebrauchsstellung den die Anode tragenden Schaft oder Schlauch nur so weit zurückzuziehen, dass der an dem ersten Schaft befindliche Anker in das Herzgewebe eingebracht werden kann, das Isolierstück aber die Herzwand innenseitig berührt, um eine bestmögliche Reizleitung zwischen den Polen, also zwischen Anode und Kathode, durch das reizfähige Herzgewebe zu gewährleisten und Verluste in diesem Bereich so klein wie möglich zu halten.

Die relative Verschiebbarkeit der beiden Pole aufgrund der relativen Verschiebbarkeit der beiden Schäfte oder Schläuche, welche diese Pole tragen, ermöglicht also einerseits den Schutz der an der Kathode befindlichen Verankerung während der Implantation und andererseits eine für den Fluss des Reizstromes bestmögliche Anordnung in Gebrauchsstellung.

Der die Anode tragende zweite Schaft oder Schlauch kann an seinem proximalen Ende eine Kupplung aufweisen, die relativ zu dem innenliegenden ersten Schaft verschiebbar und festlegbar oder fixierbar ist. Diese Fixierung könnte dabei zunächst schon während des Vorgangs der Implantation bestehen, dann für die Relativverschiebung der beiden Schäfte gegeneinander gelöst und in der verschobenen Position erneut geschlossen werden. Dabei kann der zweite Schaft im Bereich der Kupplung mittels einer lösbaren Klemmschraube fixierbar sein, was eine besonders einfache Art der Fixierung darstellt.

Ferner ist es möglich, das die wenigstens eine Klemmschraube gleichzeitig die elektrische Verbindung zwischen der Anode und dem bipolaren Stecker oder einem zu diesem führenden Leitungsstück kontaktiert. Dies vereinfacht den gesamten Aufbau der Elektrode.

Eine weitere Ausgestaltungsmöglichkeit der Erfindung kann darin bestehen, dass die Kathode und die Anode durch die relative Verschiebbarkeit der sie tragenden Schäfte oder Schläuche mit einem wählbaren Abstand zueinander anordenbar sind. Es ist also möglich, dass die beiden Pole räumlich zueinander verstellbar und einstellbar sind, um sie entweder an unterschiedliche Anatomien im Bereich der Innenseite der Herzwand anpassen zu können oder aber einen optimalen gegenseitigen Abstand nach dem Verankern der Kathode herstellen zu können.

Vor allem bei Kombination einzelner oder mehrerer der vorbeschriebenen Merkmale und Maßnahmen ergibt sich eine implantierbare bipolare Elektrode, bei welcher die Verankerung, bevorzugt eine Schraubwendel, unveränderlich an dem die Kathode tragenden Schaft oder Schlauch angeordnet, während der Implantation aber dennoch abgedeckt sein kann, weil für den zweiten Pol, nämlich die Anode, ebenfalls ein Schaft oder Schlauch vorgesehen ist, der relativ zu dem ersten Schaft oder Schlauch genügend weit verschieblich ist, um während der Implantation die Verankerung an der Kathode in sich aufzunehmen. Der die Anode tragende Schaft oder Schlauch hat also eine Doppelfunktion, weil er während der Implantation im Bereich der Verankerung einen Schutz gegen Verletzungen oder Verhakungen bilden kann.

Nachstehend ist ein Ausführungsbeispiel der Erfindung anhand der Zeichnung näher beschrieben. Es zeigt in zum Teil schematisierter Darstellung:
- Fig. 1: eine Seitenansicht einer erfindungsgemäßen bipo- laren implantierbaren Elektrode, wobei ein die Anode tragender zweiter Schaft oder Schlauch rela- tiv zu einem die Kathode tragenden ersten Schaft zum distalen Ende hin verschoben ist,
- Fig. 2: einen Längsschnitt durch das Ende der Elektrode ge- mäß Fig. 1, wobei der vorgeschobene, die Anode tra- gende Schaft eine am Ende des die Kathode aufwei- senden ersten Schafts befindliche, als Schraubwen- del dienende Verankerung außenseitig umschließt und vollständig in sich aufnimmt,
- Fig. 3: eine der Fig. 1 entsprechende Darstellung nach dem Zurückziehen des die Anode tragenden Schafts rela- tiv zu dem die Kathode und die Verankerung auf- weisenden Schaft sowie
- Fig. 4: eine der Fig. 2 entsprechende Darstellung nach dem Zurückziehen des die Anode tragenden Schafts, wo- durch die als Anker dienende Schraubwendel freige- geben und freigelegt ist.

Eine im Ganzen mit 1 bezeichnete implantierbare bipolare Elektrode weist an ihrem proximalen Ende einen bipolaren Stecker 2 auf, mit welchem die Elektrode 1 an einen Herzschrittmacher angeschlossen werden kann.

An ihrem distalen Ende befindet sich die von einem ersten Schaft oder Schlauch 3 getragene Kathode 4, die dabei auch als als Anker dienende Schraubwendel 5 ausgebildet sein kann. Die Elektrode 1 ist also am distalen Ende mit einem Anker 5 zum Befestigen im Gewebe eines Herzens versehen. Ferner ist eine zu der Kathode 4 zumindest in Gebrauchsstellung beabstandete Anode 6 vorgesehen, so dass also zwei Pole 4 und 6 die Elektrode 1 bipolar machen.

Beim Vergleich aller Figuren wird deutlich, dass diese bipolare Elektrode 1 eigentlich aus zwei unipolaren Elektroden gebildet ist, deren eine an ihrem distalen Ende die Kathode 4 und den Anker 5 aufweist und deren andere an einem zweiten Schaft oder Schlauch 7 die Anode 6 aufweist oder trägt. Dabei wäre auch denkbar, zwei Kathoden 4 und/oder Anoden 6 vorzusehen.

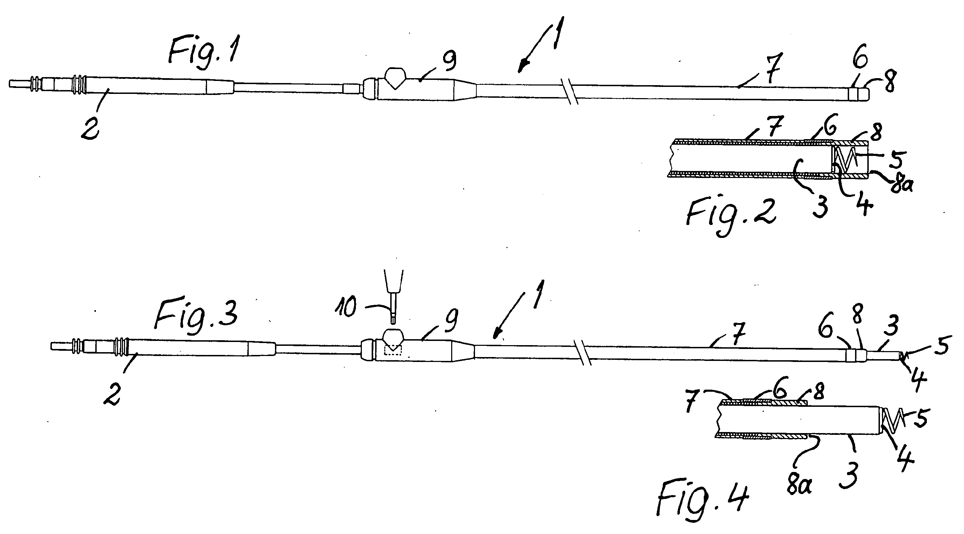

Weiterhin wird durch den Vergleich der Fig. 1 mit Fig. 3 und der Fig. 2 mit Fig. 4 deutlich, dass der zweite Schaft 7 den die Kathode 4 aufweisenden ersten Schaft oder Schlauch 3 in sich aufnimmt und/oder konzentrisch umfasst und dass diese beiden Schäfte 3 und 7 relativ zueinander in axialer Richtung verstellbar oder verschiebbar sind. Zumindest in der in Fig. 3 und 4 dargestellte Gebrauchsstellung sind dabei Kathode 4 und Anode 6 jeweils elektrisch mit dem bipolaren Stecker 2 verbunden.

Es ist also möglich, die Elektrode 1 in der in Fig. 1 dargestellten Verschiebeposition der beiden Schäfte 3 und 7 zu implantieren, ohne dass die Gefahr besteht, dass die als Anker dienende Schraubwendel 5 zu Verletzungen der Blutgefäße führt, durch welche die Implantation erfolgt. Auch Verhakungen am Eintritt in das Herz selber sind durch den die Wendel 5 in dieser Lage in sich aufnehmenden zweiten Schaft 7 vermieden.

Der die Anode 6 tragende zweite Schaft oder Schlauch 7, welcher den ersten Schaft 3 konzentrisch umschließt, ist dabei axial gegenüber dem ersten Schaft 3 um ein Maß verstellbar, das die axiale Ausdehnung der Verankerung oder der zur Verankerung dienenden Schraubwendel 5 erheblich übertrifft, so dass in der Gebrauchsstellung gemäß Fig. 3 und 4 einerseits der gewünschte Abstand von Anode und Kathode besteht und andererseits auch eine gute Verankerung sichergestellt ist.

Dabei erkennt man in den Fig. 2 und 4, dass der die Anode 6 tragende zweite Schaft oder Schlauch 7 an seinem distalen Ende benachbart zu der Anode 6 ein hülsenartiges Isolierstück 8 aufweist, welches gegenüber der Anode 6 vorsteht und eine Verlängerung des schlauchartigen Schafts 7 bilden kann. Denkbar wäre, nach dem Implantieren und Verankern den zweiten Schaft 7 wieder so weit vorzuschieben, dass das stirnseitige Ende 8a dieses Isolierstücks an der Herzwand innenseitig zur Anlage kommt, um eine bestmögliche Leitung des Reizstroms zwischen den beiden Polen zu erhalten und Verluste in diesem Bereich so klein wie möglich zu halten.

Der die Anode 6 tragende zweite Schaft oder Schlauch 7 weist an seinem proximalen Ende eine im Ganzen mit 9 bezeichnete Kupplung auf, die relativ zu dem innenliegenden ersten Schaft 3 verschiebbar und festlegbar oder fixierbar ist. Dabei ist der zweite Schaft 7 im Bereich der Kupplung 9 mittels einer lösbaren Klemmschraube fixierbar, deren Betätigung in Fig. 3 durch ein entsprechendes Werkzeug 10 angedeutet ist. Bei gelöster Klemmschraube kann also die relative Verschiebbarkeit der beiden Schäfte durchgeführt werden, die sowohl beim Implantieren, als auch in der späteren Gebrauchsstellung aber dann fixiert werden kann.

Dabei kann diese Klemmschraube gleichzeitig die elektrische Verbindung zwischen der Anode und dem bipolaren Stecker 2 oder einem zu diesem führenden Leitungsstück herstellen oder kontaktieren, so dass in Gebrauchsstellung die Pole der insgesamt bipolaren Elektrode 1 mit dem bipolaren Stecker 2 in Verbindung sind.

Die implantierbare bipolare Elektrode 1 weist an ihrem distalen Ende wenigstens zwei Pole, nämlich eine Kathode 4 und eine dazu beabstandete Anode 6 auf. Ferner ist eine beispielsweise als Schraubwendel 5 vorgesehene Verankerung am distalen Ende der Elektrode 1 vorgesehen. Die unterschiedlichen Pole sind an unterschiedlichen, relativ zueinander verschiebbaren Schäften oder Schläuchen 3 und 7 angeordnet, wobei der außenliegende Schaft oder Schlauch 7 zunächst bis über den Anker 5 vorgeschoben sein kann, um diesen beim Implantieren zu schützen. in Gebrauchsstellung sind die Pole durch eine Relativbewegung der beiden sie tragenden Schäfte hinsichtlich ihrer gegenseitigen Beabstandung eingestellt.

## Patentansprüche

1. Implantierbare bipolare Elektrode (1) mit einer an ihrem distalen Ende befindlichen, von einem Schaft oder Schlauch (3) getragenen Kathode (4) und mit einer dazu beabstandeten Anode (6), wobei die Elektrode am distalen Ende einen als Schraubwendel (5) ausgebildeten Anker zum Befestigen im Gewebe eines Herzens aufweist, wobei die bipolare Elektrode (1) aus zwei unipolaren Elektroden gebildet ist, derer eine an ihrem distalen Ende die Kathode (4) und den Anker (5) aufweist und derer andere an einem zweiten Schaft oder Schlauch die Anode (6) aufweist, wobei der zweite Schaft oder Schlauch (7) den ersten Schaft oder Schlauch (3) in sich aufnehmen kann und relativ zu diesem in axialer Richtung verstellbar oder verschiebbar ist und zumindest in Gebrauchsstellung Kathode (4) und Anode (6) jeweils elektrisch mit einem bipolaren Stecker (2) verbunden werden kann, **dadurch gekennzeichnet, dass** der zweite Schaft (7) derart ausgebildet ist, dass er den Anker während der Implantation in sich aufnehmen und umschließen kann und dass er zumindest um das Maß axial relativ zu dem ersten Schaft oder Schlauch (3) verstellbar ist, welches die Schraubwendel, (5) gegenüber dem distalen Ende des ersten Schafts oder Schlauch (3) vorsteht.

2. Elektrode nach Anspruch 1 oder 2, **dadurch gekennzeichnet, dass** der die Anode tragende zweite Schaft den die Kathode aufweisenden ersten Schaft konzentrisch umschließt.

3. Elektrode nach einem der Ansprüche 1 bis 3, **dadurch gekennzeichnet, dass** der die Anode (6) tragende zweite Schaft (7) an seinem distalen Ende benachbart zu der Anode (6) ein hülsenartiges Isolierstück (8), vorzugsweise eine Verlängerung des schlauchartigen Schafts (7) relativ zu der Anode aufweist, welches gegenüber der Anode vorsteht.

4. Elektrode nach einem der Ansprüche 1 bis 4, **dadurch gekennzeichnet, dass** der die Anode (6) tragende zweite Schaft (7) an seinem proximalen Ende eine Kupplung (9) aufweist, die relativ zu dem innenliegenden ersten Schaft (3) verschiebbar und festlegbar oder fixierbar ist.

5. Elektrode nach einem der Ansprüche 1 bis 5, **dadurch gekennzeichnet, dass** der zweite Schaft (7) im Bereich der Kupplung (9) mittels einer lösbaren Klemmschraube fixierbar ist.

6. Elektrode nach einem der Ansprüche 1 bis 6, **dadurch gekennzeichnet, dass** die wenigstens eine Klemmschraube gleichzeitig die elektrische Verbindung zwischen der Anode und dem bipolaren Stecker (2) oder einem zu diesem führenden Leitungsstück kontaktiert.

## Claims

1. Implantable bipolar electrode (1) having a cathode (4) located at its distal end, carried by a shaft or tube (3), and having an anode (6) spaced therefrom, the electrode at the distal end having an anchor in the form of a spiral coil (5) for securing in the tissue of a heart, the bipolar electrode (1) being formed by two unipolar electrodes, one of which comprises at its distal end the cathode (4) and the anchor (5) while the other comprises the anode (6) on a second shaft or tube, wherein the second shaft or tube (7) may accommodate the first shaft or tube (3) inside it and is adjustable or movable relative to the latter In the axial direction, and at least in the position of use the cathode (4) and the anode (6) may each be electrically connected to a bipolar plug (2), **characterised in that** the second shaft (7) is constructed so that it is able to accommodate and surround the anchor in the form of a spiral coil (5) during the implantation, and it is axially adjustable relative to the first shaft or tube (3) at least by the amount that the spiral coil (5) projects beyond the distal end of the first shaft or tube (3).

2. Electrode according to claim 1 or 2, **characterised in that** the second shaft carrying the anode concentrically surrounds the first shaft that comprises the cathode.

3. Electrode according to one of claims 1 to 3, **characterised in that** the second shaft (7) that carries the anode (6) comprises at its distal end, adjacent to the anode (6), a sleeve-like insulating member (8), preferably an extension of the tube-like shaft (7) relative to the anode and projecting beyond the anode.

4. Electrode according to one of claims 1 to 4, **characterised in that** the second shaft (7) carrying the anode (6) comprises, at its proximal end, a coupling (9) which is movable and securable or fixable relative to the inwardly located first shaft (3).

5. Electrode according to one of claims 1 to 5, **characterised in that** the second shaft (7) is fixable in the region of the coupling (9) by means of a releasable clamping screw.

6. Electrode according to one of claims 1 to 6, **characterised in that** the at least one clamping screw simultaneously contacts the electrical connection between the anode and the bipolar plug (2) or a lead section leading thereto.

## Revendications

1. Electrode bipolaire implantable (1) avec une cathode (4) située à son extrémité distale, portée par un corps ou tuyau (3), et avec une anode (6) distante de celle-ci, l'électrode présentant à l'extrémité distale une ancre réalisée sous la forme d'une spirale de fixation (5) pour la fixation dans le tissu d'un coeur, l'électrode bipolaire (1) étant formée de deux électrodes unipolaires dont une présente la cathode (4) et l'ancre (5) à son extrémité distale et dont l'autre présente l'anode (6) sur un deuxième corps ou tuyau, le deuxième corps ou tuyau (7) pouvant recevoir le premier corps ou tuyau (3) en son sein et étant déplaçable ou coulissant par rapport à celui-ci en direction axiale et cathode (4) et anode (6) pouvant être reliées chacune électriquement à un connecteur bipolaire (2) au moins en position d'utilisation, **caractérisée en ce que** le deuxième corps ou tuyau (7) est configuré de façon à pouvoir recevoir et renfermer en son sein l'ancre pendant l'implantation et à pouvoir être déplacé axialement par rapport au premier corps ou tuyau (3) de la mesure dont la spirale de fixation (5) est en saillie par rapport à l'extrémité distale du premier corps ou tuyau (3).

2. Electrode selon la revendication 1 ou 2, **caractérisée en ce que** le deuxième corps portant l'anode entoure concentriquement le premier corps présentant la cathode.

3. Electrode selon une des revendications 1 à 3, **caractérisée en ce que** le deuxième corps (7) portant l'anode (6) présente à son extrémité distale, au voisinage de l'anode (6), un pièce isolante (8) en forme de manchon, de préférence un prolongement du corps (7) en forme de tuyau par rapport à l'anode, lequel est en saillie par rapport à l'anode.

4. Electrode selon une des revendications 1 à 4, **caractérisée en ce que** le deuxième corps (7) portant l'anode (6) présente à son extrémité proximale un accouplement (9) qui est coulissant par rapport au premier corps (3) intérieur et qui peut être immobilisé ou fixé.

5. Electrode selon une des revendications 1 à 5, **caractérisée en ce que** le deuxième corps (7) peut être fixé au moyen d'une vis de serrage amovible dans la zone de l'accouplement (9).

6. Electrode selon une des revendications 1 à 6, **caractérisée en ce que** ladite au moins une vis de serrage contacte en même temps la liaison électrique entre l'anode et le connecteur bipolaire (2) ou une pièce de conduction menant à celui-ci.
